# EUROPÄISCHE PATENTSCHRIFT

(11) **EP 3 622 996 B1**
(45) Veröffentlichungstag und Bekanntmachung des Hinweises auf die Patenterteilung: **04.05.2022**
(21) Anmeldenummer: 18194486.9
(22) Anmeldetag: 14.09.2018
(51) Int. Cl.: A61M 16/10, A61M 16/16, A61M 15/00, A61M 11/06

(54) **GASAUFBEREITER FÜR EINE INHALIERVORRICHTUNG**
GAS PREPARATION DEVICE FOR AN INHALATION DEVICE
DISPOSITIF DE TRAITEMENT DE GAZ POUR UN DISPOSITIF INHALATEUR

(43) Veröffentlichungstag der Anmeldung: 18.03.2020
(73) Patentinhaber: PARI GmbH Spezialisten für effektive Inhalation, 82319 Starnberg (DE)
(72) Erfinder: Buchmann, Nicolas, 82008 Unterhaching (DE); Schwenck, Nicolas, 80337 München (DE); Neemann, Carsten, 26655 Westerstede (DE); Schuschnig, Uwe, 81371 München (DE); Fritz, Christoph, 85221 Dachau (DE)
(74) Vertreter: Hoffmann Eitle

(56) Entgegenhaltungen:
- EP-A1- 2 046 155
- WO-A1-2012/025846
- WO-A1-2017/109737
- WO-A1-2017/207586
- US-A1- 2016 331 027
- US-A1- 2017 035 981
- US-B2- 7 143 766

## Beschreibung

Die Erfindung betrifft ein Inhaliersystem.

Im Stand der Technik sind Vorrichtungen zur Warminhalation bekannt.

Die US 2017/0035981 A1 umfasst Masken und Systeme zur Bereitstellung von Atembehandlungen für verschiedene Tiere, einschließlich Pferde, Maultiere und Ponys.

Die Masken sind aufgrund einfacher Konstruktionen, einer sehr geringen Dicke, die weniger Material erfordert, kostengünstig und die Systeme umfassen die Masken der Erfindung.

Die WO 2017/207586 A1 umfasst ein elektrisch betriebenes Aerosolerzeugungssystem, umfassend einen Aerosolerzeugungsgegenstand und eine elektrisch betriebene Aerosolerzeugungsvorrichtung. Der Aerosolerzeugende Gegenstand umfasst ein aerosolbildendes Substrat und hat ein Mundende und ein distales Ende stromaufwärts vom Mundende. Die elektrisch betriebene Aerosolerzeugungsvorrichtung umfasst ein Gehäuse mit einem Hohlraum, der konfiguriert ist, um das distale Ende des Aerosolerzeugungsartikels aufzunehmen. Das Aerosolerzeugungssystem umfasst ferner ein elektrisches Heizelement und einen Wärmediffusor, der einen nicht brennbaren porösen Körper umfasst, um Wärme von dem elektrischen Heizelement so aufzunehmen, dass im Gebrauch Luft angesaugt wird durch den Aerosol erzeugenden Gegenstand vom distalen Ende zum Mundende wird durch die im porösen Körper absorbierte Wärme erwärmt.

DE4225928A1 beschreibt eine Vorrichtung zum Zerstäuben, Verteilen und Vermischen von flüssigen oder pulverförmigen Stoffen mittels eines Druckgasstromes, insbesondere für die Erzeugung von Aerosolen für Inhalationszwecke, mit einer Zerstäubungsdüse in einem Zerstäubungsraum in den ein Zuluftkamin für den Eintritt von Zuluft hineinragt. Im Zuluftkamin ist eine elektrische Heizeinrichtung angeordnet, mit deren Hilfe die einströmende Zuluft erwärmt wird.

Eine weitere bisher bekannte Ausgestaltung eines Gasaufbereiters geht aus der WO 2012 025 846 A1 hervor, welche die Grundlage für den Oberbegriff der zweiteiligen Fassung von Anspruch 1 bildet.

Aufgabe der vorliegenden Erfindung ist es, eine Alternative bereitzustellen.

Die Aufgabe wird gelöst durch ein Inhaliersystem mit einer Inhaliervorrichtung und einem Gasaufbereiter für die Inhaliervorrichtung gemäß Anspruch 1 und/oder die Verwendung dieses Inhaliersystems zur Bereitstellung einer Vorrichtung für eine Warminhalation gemäß Anspruch 8. Weitere Ausführungsformen ergeben sich aus den abhängigen Ansprüchen.

In dem Inhaliersystem mit der Inhaliervorrichtung und dem Gasaufbereiter für die Inhaliervorrichtung weist der Gasaufbereiter einen Gasaufbereitungsbereich, einen Wärmespeicher, eine Gaszuführung und einen Inhaliervorrichtungsanschluss auf, wobei der Gasaufbereitungsbereich einen Wärmeübertragungsbereich aufweist, der Wärmespeicher dazu eingerichtet ist, in einer Aufladephase Energie von einer Ladestation aufzunehmen und in einer Inhalationsphase die in der Aufladephase aufgenommene Energie oder einen Teil davon in Form von Wärme abzugeben, der Wärmeübertragungsbereich mit dem Wärmespeicher thermisch verbunden ist, die Gaszuführung dazu eingerichtet ist, ein Gas in den Gasaufbereitungsbereich zu führen und der Inhaliervorrichtungsanschluss eine durchströmbare Verbindung zu dem Gasaufbereitungsbereich aufweist und durchströmbar mit einer Inhaliervorrichtung verbindbar oder verbunden ist.

Die Inhaliervorrichtung weist einen Gasaufbereiteranschluss auf, der mit dem Inhaliervorrichtungsanschluss durchströmbar verbindbar oder verbunden ist.

Ferner weist das Inhaliersystem einen Bypass und eine Steuereinrichtung auf, wobei der Bypass dazu eingerichtet ist, eine Verbindung zwischen der Inhaliervorrichtung und einem Gasvolumen bereitzustellen und die Steuereinrichtung dazu eingerichtet ist, ein Verhältnis zwischen einem Bypassströmungsquerschnitt und einem Gasaufbereiterströmungsquerschnitt einzustellen.

Eine für die Inhalation angenehme Aerosoltemperatur kann so auf einfache Weise bereitgestellt werden.

Der Gasaufbereiter ist dazu eingerichtet, eine Zulufterwärmung einer Inhaliervorrichtung zu ermöglichen. Zuluft, die im Betrieb der Inhaliervorrichtung oder des Verneblers durch den Vernebler strömt oder in den Vernebler eingebracht wird, kann so auf einfache Weise erwärmt werden. Eine Erwärmung des Aerosols kann durch Erwärmung der Zuluft erreicht werden. In einer Ausführungsform ist der Gasaufbereiter als Baugruppe vorgesehen. Die einzelnen Bauteile sind dabei nicht dazu vorgesehen, im üblichen Gebrauch voneinander getrennt zu werden.

Vorzugsweise ist die Inhaliervorrichtung eine Aerosolerzeugungsvorrichtung. Eine Aerosolerzeugungsvorrichtung weist bevorzugt einen Vernebler, einen Zerstäuber, einen Befeuchter, einen Druckluftvernebler, einen Luftzerstäuber, einen elektronischen Vernebler, einen Ultraschallvernebler, einen elektrohydrodynamischen Vernebler, einen elektrostatischen Vernebler, einen Membranvernebler, einen Vernebler mit einer vibrierenden Membran, einen elektronischen Vernebler mit einer vibrierenden Membran, einen Mesh-Vernebler, einen Düsenvernebler, einen Inhalator (MDI), einen Pulverzerstäuber (DPI) oder eine Kombination davon auf. Der Inhalator weist in einer Ausführungsform einen unter Druck stehenden Kanister mit einem Medikament und einem Treibgas auf. Zweckmäßigerweise ist der Kanister mit einem von Hand bedienbaren Aktuator verbunden. Es ist vorteilhaft, wenn der Inhalator eingerichtet ist, bei der Aktivierung eine bestimmte Medikamentenmenge in Aerosolform abzugeben. In einer Ausführungsform ist die Aerosolerzeugungsvorrichtung für den Einsatz mit Beatmungsvorrichtungen eingerichtet.

Es ist vorteilhaft, wenn die Inhaliervorrichtung eine Vorrichtung zur Bereitstellung von Aerosolen ist. Die Vorrichtung zur Bereitstellung von Aerosolen weist bevorzugt eine Inhalierhilfe, einen Spacer oder eine Chamber auf. Vorrichtungen zur Bereitstellung von Aerosolen sind vorzugsweise Vorrichtungen, die zur Verwendung mit Inhalatoren (MDIs) vorgesehen sind. Sie stellen Medikamentenräume bereit, die dazu eingerichtet sind, Aerosol bevorzugt aus Inhalatoren aufzunehmen, so dass es von Benutzern daraus abgeatmet werden kann. Spacer weisen keine Ein- und Ausatemventile auf, so dass ein Benutzer seine Atmung so koordinieren sollte, dass er nicht in den Spacer ausatmet. Chambers oder Holding Chambers weisen Aus- und bevorzugt auch Einatemventile auf. So kann erreicht werden, dass ein Ausatemstrom nicht in den Raum, in dem sich das Aerosol befindet, geleitet wird. Es kann erreicht werden, dass ein Medikament nur beim Einatmen aus dem Medikamentenraum heraus gelangen kann.

Aerosole sind Gemische aus festen oder flüssigen Schwebeteilchen und einem Gas. Aerosole sind bevorzugt zur Applikation auf oder in Teile des menschlichen oder tierischen Körpers, wie Haut, Körperhöhlen, Körperöffnungen, Nase, Nasennebenhöhlen, Kieferhöhle, Stirnhöhle, Keilbeinhöhle, Siebbeinzellen, Rachen, Kehlkopf, Luftröhre, Lunge, Stammbronchus, Bronchien, Bronchiolen, Lungenbläschen, Gelenke oder Bauchraum vorgesehen. Aerosole können eingesetzt werden, um Krankheiten von Menschen und Tieren zu diagnostizieren, vorzubeugen, zu therapieren oder um Menschen oder Tiere gegen Krankheiten zu immunisieren.

Ein Gasaufbereitungsbereich ist ein Bereich, der dazu vorgesehen ist, eine oder mehrere Eigenschaften eines Gases zu verändern.

Ein Wärmespeicher ist eine Vorrichtung, die dazu eingerichtet ist, Energie, vorzugsweise Wärmeenergie, besonders bevorzugt Wärme speichern und abgeben zu können. Es ist zweckmäßig, wenn der Wärmespeicher dazu eingerichtet ist, Energie, bevorzugt Wärmeenergie oder Wärme aufnehmen zu können. Der Wärmespeicher ist vorzugsweise dazu eingerichtet, in einer Aufladephase Energie aufzunehmen und in einer Inhalationsphase Wärme abzugeben. Es ist besonders zweckmäßig, wenn der Wärmespeicher dazu eingerichtet ist, während der Inhalationsphase keine Energie aufzunehmen. Vorzugsweise ist der Wärmespeicher dazu eingerichtet, während der Inhalationsphase unbestromt zu sein. Vorteile können erzielt werden, wenn der Gasaufbereiter dazu eingerichtet ist, während der Inhalationsphase unbestromt zu sein. Vorzugsweise ist der Wärmetauscher funktional, räumlich oder auf beide Weisen mit einer Ladestation verbindbar und trennbar. Zweckmäßigerweise ist der Wärmetauscher dazu eingerichtet, während einer Aufladephase mit der Ladestation funktional, räumlich oder auf beide Weisen verbunden zu sein und während einer Inhalationsphase funktional, räumlich oder auf beide Weisen getrennt zu sein. Auf diese Weise kann ein Gasaufbereiter bereitgestellt werden, der dem Anwender mit einfachen Maßnahmen eine hohe Sicherheit bieten kann.

Der Wärmespeicher kann fest mit den anderen Bestandteilen des Gasaufbereiters verbunden sein. In einer Ausführungsform ist der Wärmespeicher derart ausgestaltet, dass er von den übrigen Bestandteilen des Gasaufbereiters lösbar ist. Der Gasaufbereiter kann fest mit den anderen Bestandteilen des Inhaliersystems verbunden sein. Vorteile können erzielt werden, wenn der Gasaufbereiter in die Inhaliervorrichtung integriert ist. Der Gasaufbereiter kann derart ausgestaltet sein, dass er von den übrigen Bestandteilen des Inhaliersystems lösbar ist. Der Wärmespeicher ist separat, zusammen mit den übrigen Bestandteilen des Gasaufbereiters, zusammen mit den übrigen Bestandteilen des Inhaliersystems oder auf mehrere dieser Arten aufladbar.

In einer bevorzugten Ausführungsform ist der Wärmespeicher dazu geeignet, während der Inhalationsphase, während welcher dem Wärmespeicher keine weitere externe Energie zugeführt wird, an einen Zuluftstrom für eine Dauer von fünf Sekunden bis 20 Minuten, vorzugsweise für eine Dauer von 10 Sekunden bis 10 Minuten, besonders bevorzugt für eine Dauer von einer Minute bis fünf Minuten eine thermische Leistung von 3 Watt bis 40 Watt, vorzugsweise von 5 Watt bis 25 Watt, besonders bevorzugt von 6 Watt bis 20 Watt abzugeben. In einer Ausführungsform ist der Wärmespeicher dazu geeignet, während der Inhalationsphase, während welcher dem Wärmespeicher keine weitere externe Energie zugeführt wird, für eine Dauer von fünf Sekunden bis 20 Minuten, vorzugsweise für eine Dauer von 10 Sekunden bis 10 Minuten, besonders bevorzugt für eine Dauer von einer Minute bis fünf Minuten eine thermische Leistung von 12 Watt bis 30 Watt an einen Zuluftstrom abzugeben.

Der Wärmespeicher weist zweckmäßigerweise ein Material mit entsprechend hoher Wärmekapazität zur Speicherung von thermischer Energie auf. Vorzugsweise ist eine Oberfläche des Wärmespeichers als Wärmeübertragungsbereich ausgebildet. Zweckmäßigerweise ist die Oberfläche zur Übertragung von thermischer Energie auf einen Luftstrom ausgeformt und optimiert.

Der Wärmespeicher ist in einer Ausführungsform als Latentwärmespeicher ausgebildet. Vorzugsweise ist der Wärmespeicher als thermochemischer Wärmespeicher, besonders bevorzugt als sorptiver Wärmespeicher oder Sorptionswärmespeicher ausgebildet.

Ein sorptiver Wärmespeicher weist ein Adsorbens auf, das bevorzugt ein Metal Organic Framework, Aktivkohle, ein Zeolith, ein Silikagel, ein Metallhydrid oder eine Kombination daraus aufweist. Das Metal Organic Framework weist vorzugsweise MOF-5 auf. Die Aktivkohle ist zweckmäßigerweise modifiziert und hydrophil. Das Zeolith weist vorzugsweise Zeolith A, Zeolith X, Zeolith Y, Zeolith L, Mordenit, ZSM5, ZSM 11 oder eine Kombination daraus auf.

Der sorptive Wärmespeicher ist vorzugsweise dazu vorgesehen, mit einem Adsorptiv betrieben zu werden, das Wasser, Wasserdampf, Aerosol, Methanol oder eine Kombination daraus aufweist.

Der Wärmespeicher ist in einer Ausführungsform als flüssiger Wärmespeicher ausgebildet. Bevorzugt ist der Wärmespeicher als Feststoffwärmespeicher ausgebildet.

Ein Feststoffwärmespeicher ist ein Wärmespeicher, der einen Feststoff, vorzugsweise Ton, Silikagel, Keramik, Kerne, Körner, Kirschkerne, besonders bevorzugt ein Zeolith aufweist.

Vorzugsweise weist der Wärmespeicher ein poröses Material und eine oberflächenoptimierte, große Oberfläche auf.

In einer Ausführungsform weist der Wärmespeicher Elemente auf, die zu einem porösen Paket zusammengefasst sind. Vorzugsweise weisen die Elemente Durchmesser im Bereich von 0,1 bis 10 Millimeter auf. In einer Ausführungsform weist der Wärmespeicher Elemente in Kugelform auf. Vorzugsweise weist der Wärmespeicher unregelmäßig geformte Elemente auf.

In einer Ausführungsform weist der Wärmespeicher eine Aufnahme für Eisenpulver auf. Diese Ausführungsform ist dazu eingerichtet, in einer Aufladephase mit Eisenpulver bestückt zu werden, das dazu vorgesehen ist, in der Inhalationsphase mit Sauerstoff zu reagieren und Wärme freizusetzen.

Ein flüssiger Wärmespeicher ist ein Wärmespeicher, der ein flüssiges Medium, vorzugsweise Wasser oder Öl aufweist.

Ein Latentwärmespeicher ist ein Wärmespeicher, der ein Medium aufweist, welches bei Erwärmung oder Abkühlung einen Phasenübergang aufweist. Der Phasenübergang findet vorzugsweise zwischen fest und flüssig statt. In einer Ausführungsform weist der Latentwärmespeicher ein Paraffin, Wachs oder Salz auf.

Der Wärmespeicher weist vorzugsweise optimierte Oberflächen für einen maximal effizienten Wärmetransport auf. Vorzugsweise weist der Wärmespeicher ein Rohrbündel oder ein Laminar-Fluss-Element auf.

Es ist besonders vorteilhaft, wenn die Energiespeicherung reversibel ist und ohne signifikante Reduktion der thermischen Kapazität des Wärmespeichers beliebig oft wiederholt werden kann.

Es ist bevorzugt, wenn der Wärmespeicher portabel, autonom, aufladbar, wiederaufladbar, wiederverwendbar oder passiv ausgestaltet ist oder eine Kombination dieser Eigenschaften aufweist.

Vorzugsweise ist der Wärmespeicher dazu ausgelegt, durch Mikrowellen, Kochen, Erwärmen, zweckmäßigerweise in einem Backofen, chemische Reaktion, Induktion, Solarenergie oder eine Kombination daraus aufgeladen zu werden. In einer Ausführungsform ist der Wärmespeicher dazu ausgelegt, durch eine Reaktion von Eisenpulver mit Sauerstoff aufgeladen zu werden. In einer Ausführungsform ist der Wärmespeicher dazu ausgelegt, durch einen Phasenübergang eines Latentwärmespeichers aufgeladen zu werden.

In einer Ausführungsform ist der Wärmespeicher dazu vorgesehen, von einer Verneblerzuluft oder einem Luftstrom ganz oder teilweise durchströmt, umströmt oder sowohl durchströmt als auch umströmt zu werden.

Es ist vorteilhaft, wenn der Wärmespeicher auf einen Atemfluss, eine Aerosolmenge oder beides abgestimmt ist. Bei geeigneter Auslegung des Wärmespeichers können Aerosoltemperaturen von 30°C und mehr erreicht werden. In einer Ausführungsform ist die Aerosoltemperatur durch Variation der thermischen Masse gezielt steuerbar. In einer Ausführungsform unterliegt die Aerosoltemperatur einer zeitlichen Schwankung. Die Aerosoltemperatur ist in einer Ausführungsform abhängig von der Charakteristik des Wärmespeichers. In einer Ausführungsform werden für einen Zeitraum von 5 Sekunden bis 20 Minuten, vorzugsweise für einen Zeitraum von 10 Sekunden bis 10 Minuten, besonders bevorzugt für einen Zeitraum von ein bis zwei Minuten Aerosoltemperaturen von 30°C bis 50° C erreicht.

Zweckmäßigerweise ist der Wärmespeicher dazu eingerichtet, im Vernebelungsbetrieb ohne elektrische Energie betrieben zu werden.

Der Wärmeübertragungsbereich ist ein Bereich, der dazu eingerichtet ist, Wärme zwischen dem Wärmespeicher und einem Gas zu übertragen, vorzugsweise dem Gas Wärmeenergie von dem Wärmespeicher zuzuführen. Dazu weist der Wärmeübertragungsbereich zweckmäßigerweise eine gute thermische Verbindung zu dem Wärmespeicher auf. Vorzugsweise weist der Wärmeübertragungsbereich eine Oberfläche des Wärmespeichers auf. Um die Wärme besonders gut auf das Gas übertragen zu können, weist der Wärmeübertragungsbereich vorzugsweise eine große Oberfläche auf. Die Oberfläche ist besonders bevorzugt in Form von Rippen, als Oberfläche eines porösen Stoffs, als Oberfläche einzelner Elemente oder als Kombination daraus ausgestaltet.

Der Inhaliervorrichtungsanschluss ist zweckmäßigerweise dazu eingerichtet, derart mit einer Inhaliervorrichtung verbunden zu werden, dass Gas durch den Inhaliervorrichtungsanschluss in die Inhaliervorrichtung strömen kann.

Es ist vorteilhaft, wenn der Gasaufbereiter einen Flüssigkeitsspeicher und der Gasaufbereitungsbereich einen Befeuchtungsbereich aufweist, und der Flüssigkeitsspeicher mit dem Befeuchtungsbereich verbunden ist.

Durch Befeuchtung der Zuluft kann die technische Wirkleistung optimiert werden.

Der Flüssigkeitsspeicher oder Feuchtespeicher ist dazu geeignet, Flüssigkeit oder Feuchte zu speichern. Vorzugsweise weist der Flüssigkeitsspeicher eine geeignete Oberfläche auf, die dazu eingerichtet ist, Flüssigkeit oder Feuchte an ein durch-, umströmendes oder sowohl durchströmendes als auch umströmendes Gas, vorzugsweise Zuluft abzugeben.

Der Flüssigkeitsspeicher weist vorzugsweise ein poröses Material und eine optimierte Oberfläche auf. In einer Ausführungsform weist der Feuchtespeicher einen porösen Feststoff zur Speicherung von Flüssigkeit oder Feuchtigkeit, vorzugsweise Tongranulat, Keramik, Zellulose oder einen Schwamm auf.

In einer Ausführungsform weist der Flüssigkeitsspeicher ein flüssiges Medium, vorzugsweise Wasser auf.

In einer Ausführungsform ist der Flüssigkeitsspeicher dazu geeignet, eine Flüssigkeit bei Erwärmung verdunsten oder verdampfen zu lassen. Vorzugsweise ist der Feuchtespeicher dazu optimiert, Wasser bei Erwärmung als gasförmiges Wasser abzugeben.

Der Befeuchtungsbereich ist ein Bereich, der dazu eingerichtet ist, ein Gas, vorzugsweise Luft, zu befeuchten. Zweckmäßigerweise weist der Befeuchtungsbereich eine große Oberfläche auf, die dazu eingerichtet ist, von einem Gas überstrichen zu werden. Vorzugsweise weist der Befeuchtungsbereich die Oberfläche eines porösen Feststoffs, eines Tongranulats, einer Keramik, von Zellulose oder eines Schwamms auf. Besonders bevorzugt weist der Befeuchtungsbereich eine Oberfläche des Flüssigkeitsspeichers auf.

Der Flüssigkeitsspeicher ist mit dem Befeuchtungsbereich zweckmäßigerweise derart verbunden, dass Flüssigkeit gut aus dem Flüssigkeitsspeicher in den Befeuchtungsbereich gelangen kann.

In einer Ausführungsform ist der Befeuchtungsbereich stromaufwärts des Wärmeübertragungsbereichs angeordnet. Auf diese Weise kann dem Gas Wärme auf einfache Weise zugeführt werden.

Es ist vorteilhaft, wenn der Flüssigkeitsspeicher in den Wärmespeicher integriert ist. So kann auf besonders einfache Weise eine optimale Aerosoltemperatur erreicht werden.

Vorzugsweise ist der Flüssigkeitsspeicher in den Wärmespeicher integriert, indem ein Kombinationsspeicher für Wärme und Feuchte vorgesehen ist. Der Kombinationsspeicher ist zweckmäßigerweise zur gleichzeitigen Speicherung von Wärme und Flüssigkeit, vorzugsweise Wasser, eingerichtet.

In einer Ausführungsform weist der Kombinationsspeicher poröse Keramik, poröses Tongranulat oder eine Kombination daraus auf. Bevorzugt ist der Kombinationsspeicher oberflächenoptimiert.

In einer Ausführungsform sind Wärmeübergang, Feuchtespeicherung und Feuchtetransport durch unterschiedliche Porositäten unabhängig voneinander optimiert.

Vorzugsweise weisen Wärmeübertragungsbereich, Befeuchtungsbereich oder beide eine Oberfläche des Kombinationsspeichers auf.

In einer Ausführungsform weist der Wärmespeicher ein magnetisches Material auf. Dadurch kann der Wärmespeicher auf einfache Weise basierend auf der induktiven Wärmeerzeugung aufgeladen werden.

Der Wärmespeicher weist vorzugsweise ein Metall auf.

In einer Ausführungsform weist der Wärmespeicher ein poröses Material auf, weist der Flüssigkeitsspeicher ein poröses Material auf oder weisen sowohl der Wärmespeicher als auch der Flüssigkeitsspeicher ein poröses Material auf. Flüssigkeit kann so auf besonders einfache Weise gespeichert werden.

Es ist besonders zweckmäßig, wenn das poröse Material einen offenporigen Bereich aufweist.

In einer Ausführungsform ist das Inhaliersystem als Baugruppe vorgesehen. Die einzelnen Bauteile sind dabei nicht dazu vorgesehen, im üblichen Gebrauch voneinander getrennt zu werden.

Vorzugsweise ist die Inhaliervorrichtung als mobiles System ausgebildet, das kein zusätzliches Netzgerät oder elektrische Leitungen, die von der Inhaliervorrichtung wegführen, aufweist. In einer Ausführungsform weist das System eine stromunabhängige Aerosolerwärmung auf. Es ist besonders vorteilhaft, wenn das System derart ausgebildet ist, dass es keine elektrischen Anschlüsse zur Zulufterwärmung aufweist.

Durch die Kombination der Inhaliervorrichtung mit dem Gasaufbereiter kann eine Vorrichtung zur Warmaerosolinhalation bereitgestellt werden, die auf einfache Weise konstruiert ist, eine einfache Abdichtung aufweist, klein ist, ein geringes Gewicht und Bauvolumen hat und auf einfache Weise Sicherheit gegen Stromunfälle bereitstellen kann, da keine elektrischen Teile zur Wärmebereitstellung benötigt werden.

Der Bypass weist einen Strömungsweg zwischen dem Gasvolumen und der Inhaliervorrichtung auf, wobei der Strömungsweg den Gasaufbereiter umgeht. Dadurch, dass der Strömungsweg nicht durch den Gasaufbereiter führt, werden Temperatur und Feuchte eines durch den Bypass geleiteten Gases durch den Gasaufbereiter nicht verändert.

Durch Einstellung des Verhältnisses des Gasflusses, der durch den Gasaufbereiter geleitet wird, zu dem Gasfluss, der durch den Bypass geleitet wird, können Temperatur und Feuchte des Aerosols beeinflusst werden.

Das Gasvolumen ist vorzugsweise der Inhalt einer Gasflasche, eines zentralen Reservoirs oder eines Gasversorgungssystems, besonders bevorzugt Umgebungsluft.

Der Bypassströmungsquerschnitt ist ein vorzugsweise größenverstellbarer Querschnitt des Bypasses, stromaufwärts des Bypasses oder zwischen Bypass und Inhaliervorrichtung.

Der Gasaufbereiterströmungsquerschnitt ist ein vorzugsweise größenverstellbarer Querschnitt des Gasaufbereiters, stromaufwärts des Gasaufbereiters oder zwischen Gasaufbereiter und Inhaliervorrichtung.

Vorzugsweise weist ein Ventil den Bypassströmungsquerschnitt, den Gasaufbereiterströmungsquerschnitt oder beide auf. Zweckmäßigerweise ist der Gasaufbereiterströmungsquerschnitt, der Bypassströmungsquerschnitt oder sind beide derart größenverstellbar, dass eine vollständige Gasdichtheit eingestellt werden kann.

Die Steuereinrichtung umfasst vorzugsweise ein manuell betätigbares Ventil. In einer Ausführungsform ist das Ventil elektromotorisch, elektromagnetisch oder durch ein Medium betätigbar. Es kann vorteilhaft sein, wenn das Ventil hydraulisch oder pneumatisch betätigbar ist.

In einer Ausführungsform ist ein Wärmespeicher oder Kombinationsspeicher mit adaptiver Durchströmung und mechanischer oder elektromechanischer Temperaturregelung vorgesehen. Bevorzugt ist der Wärmespeicher oder Kombinationsspeicher mit variabler, einstellbarer oder sowohl variabler als auch einstellbarer Durchströmung zur Regulierung einer Aerosoltemperatur vorgesehen. Vorzugsweise ist die Strömung derart regelbar, dass der Wärmespeicher ganz, teilweise oder gar nicht durchströmt wird. In einer Ausführungsform ist ein Temperaturregler bereitgestellt, der auf mechanischen Prinzipien basiert. Vorzugsweise erfolgt die Einstellung der Durchströmung durch den Anwender oder Patienten. In einer Ausführungsform ist ein Temperaturregler bereitgestellt, der auf elektromechanischen Prinzipien basiert.

Es ist vorteilhaft, wenn das Inhaliersystem einen Temperatursensor umfasst, der dazu eingerichtet ist, eine Temperatur des Aerosols zu erfassen und ein Temperatursignal an die Steuereinrichtung zu leiten, die Steuereinrichtung dazu eingerichtet ist, das Temperatursignal zu empfangen und basierend auf einer Solltemperatur und dem Temperatursignal ein Verhältnis zwischen Bypassströmungsquerschnitt und Gasaufbereiterströmungsquerschnitt einzustellen.

Es ist zweckmäßig, wenn die Steuereinrichtung eingerichtet ist, Temperaturdaten zu empfangen und ein Ventil abhängig von den empfangenen Temperaturdaten einzustellen. Vorzugsweise ist die Durchströmung mit einem elektrischen Schaltventil oder Stellmotor gesteuert, das oder der an einen Temperatursensor gekoppelt ist. Der Temperatursensor weist vorzugsweise ein Bimetall, einen Thermistor oder einen NTC Thermistor auf. Zweckmäßigerweise ist die elektronische Steuerung für den Niedervoltbereich ausgelegt.

Es können Vorteile erreicht werden, wenn eine Ladestation für einen Wärmespeicher bereitgestellt wird. Zweckmäßigerweise ist die Ladestation eine separate Baugruppe. Vorzugsweise nutzt die Ladestation Wärme, Mikrowellen, eine chemische Reaktion, eine Reaktion von Eisenpulver mit Sauerstoff, einen Phasenübergang eines Latentwärmespeichers, Solarstrahlung, das Prinzip der Induktiven Erwärmung oder eine Kombination daraus. In einer bevorzugten Ausführungsform weist die Ladestation ein Wasserbad, einen Backofen oder eine Mikrowelle auf.

Vorzugsweise weist eine Ladeeinrichtung für einen Gasaufbereiter eine Spule, die dazu eingerichtet ist, von Wechselstrom durchflossen zu werden und ein magnetisches Wechselfeld zu erzeugen, das dazu geeignet ist, den Wärmespeicher zu erwärmen, auf. So kann der Gasaufbereiter auf besonders komfortable Weise mit Wärmeenergie aufgeladen werden. Eine Spule weist Wicklungen oder Wickelgüter auf.

Die Erfindung betrifft auch die Verwendung des Inhaliersystems zur Bereitstellung einer Vorrichtung für eine Warminhalation, wobei der Wärmespeicher aufgeladen wird und der

Inhaliervorrichtungsanschluss mit einem Gasaufbereiteranschluss einer Inhaliervorrichtung verbunden wird.

Bei der Verwendung der Vorrichtung für die Warminhalation wird der Wärmespeicher in einer Ausführungsform während der Einatmung durchströmt und es wird ein Teil der gespeicherten Wärme an die Einatemluft übertragen. Dadurch kommt es zu einer Aufwärmung der Einatemluft und des Aerosols. Bei dieser Ausführungsform kann der Wärmespeicher nach Beendigung der Inhalation erneut aufgeladen und für eine erneute Therapie verwendet werden.

Bei einer bevorzugten Verwendung wird in einer Aufladephase der Wärmespeicher mit Energie aufgeladen, nach der Aufladephase wird die Energieübertragung gestoppt und in einer Inhalationsphase überträgt der Wärmespeicher über den Wärmeübertragungsbereich Wärme auf das Gas

Im Folgenden wird die Erfindung anhand von Ausführungsbeispielen unter Bezugnahme auf die beigefügten Figuren genauer beschrieben.
Figur 1 zeigt einen Gasaufbereiter mit einem Wärmespeicher, der Zeolith aufweist,
Figur 2 zeigt einen Gasaufbereiter mit einem Wärmespeicher, der ein Keramikbauteil aufweist,
Figur 3 zeigt einen Gasaufbereiter mit einem Wärmespeicher, der ein Metallbauteil aufweist,
Figur 4 zeigt einen Gasaufbereiter mit einem Wärmespeicher und einem Flüssigkeitsspeicher,
Figur 5 zeigt einen Gasaufbereiter mit einem Wärmespeicher, der eine Flüssigkeit aufweist und einem Flüssigkeitsspeicher,
Figur 6 zeigt einen Gasaufbereiter mit einem Wärmespeicher, der ein Metall aufweist und einem Flüssigkeitsspeicher,
Figur 7 zeigt einen Gasaufbereiter mit einem Kombinationsspeicher für Wärme und Flüssigkeit, der ein Keramikbauteil aufweist,
Figur 8 zeigt einen Gasaufbereiter mit einem Kombinationsspeicher für Wärme und Flüssigkeit, der Kieselgel aufweist,
Figur 9 zeigt einen Gasaufbereiter mit einem Kombinationsspeicher für Wärme und Flüssigkeit, der Wasser aufweist,
Figur 10 zeigt das Schema eines Gasaufbereiters mit einem Wärmespeicher,
Figur 11 zeigt das Schema eines Gasaufbereiters mit einem Wärmespeicher und einem Flüssigkeitsspeicher,
Figur 12 zeigt das Schema eines Gasaufbereiters mit einem kombinierten Wärme- und Flüssigkeitsspeicher,
Figur 13 zeigt einen Düsenvernebler mit einem Gasaufbereiter, der einen Wärmespeicher aufweist,
Figur 14 zeigt einen Meshvernebler mit einem Gasaufbereiter, der einen Wärmespeicher aufweist,
Figur 15 zeigt einen Düsenvernebler mit einem Gasaufbereiter, der einen Wärmespeicher und einen Flüssigkeitsspeicher aufweist,
Figur 16 zeigt einen Meshvernebler mit einem Gasaufbereiter, der einen Wärmespeicher und einen Flüssigkeitsspeicher aufweist
Figur 17 zeigt einen Düsenvernebler mit einem Gasaufbereiter, der einen Kombinationsspeicher für Wärme und Flüssigkeit aufweist,
Figur 18 zeigt einen Meshvernebler mit einem Gasaufbereiter, der einen Kombinationsspeicher für Wärme und Flüssigkeit aufweist,
Figur 19 zeigt einen Düsenvernebler mit einem Gasaufbereiter und einer Temperatursteuerung,
Figur 20 zeigt einen Meshvernebler mit einem Gasaufbereiter und einer Temperatursteuerung,
Figur 21 zeigt ein Diagramm mit Temperaturverläufen.

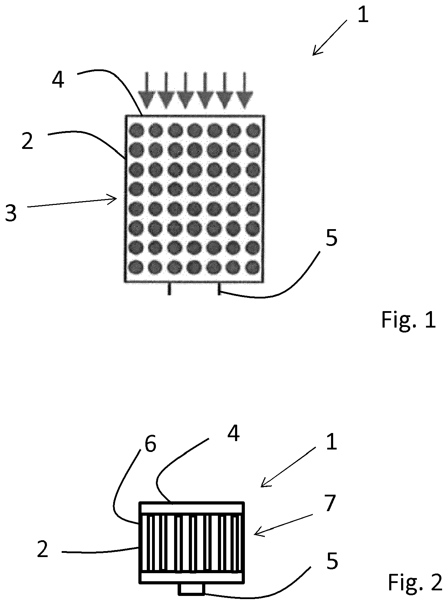

Figur 1 zeigt einen Gasaufbereiter 1 mit einem Wärmespeicher 2, der Zeolithteilchen 3 aufweist. An den Oberflächen der Zeolithteilchen 3 sind Wärmeübertragungsbereiche vorhanden. Der Gasaufbereiter 1 weist eine Gaszuführung 4 auf, die als Lochplatte ausgebildet ist. An einer der Gaszuführung 4 gegenüberliegenden Seite ist ein Inhaliervorrichtungsanschluss 5 vorgesehen.

Im Betrieb des Gasaufbereiters 1 strömt Umgebungsluft oder ein mit Überdruck beaufschlagtes Gas durch die Gaszuführung 4 in das Innere des aufgeladenen Gasaufbereiters 1. Die Umgebungsluft kann durch einen Einatemstrom durch den Gasaufbereiter 1 gesaugt werden. In dem Gasaufbereiter 1 strömt die Umgebungsluft oder das Gas an den Zeolithteilchen 3 vorbei und nimmt dabei über die Oberflächen der Zeolithteilchen 3 Wärme auf. Durch den Inhaliervorrichtungsanschluss 5 tritt erwärmtes Gas oder Umgebungsluft aus.

Figur 2 zeigt einen Gasaufbereiter 1 mit einem Wärmespeicher 2, der ein Keramikbauteil 6 aufweist. Das hier gezeigte Keramikbauteil 6 ist nicht elektrisch leitfähig. Das Keramikbauteil ist zur besseren Durchströmbarkeit und für eine bessere Wärmeübertragbarkeit mit Kanälen 7 durchzogen. Der wesentliche Unterschied des in Figur 2 gezeigten Gasaufbereiters 1 zu dem in Figur 1 gezeigten Gasaufbereiter 1 besteht darin, dass statt der Zeolithteilchen 3 ein Keramikbauteil 6 vorgesehen ist.

Die Funktion des Gasaufbereiters 1 entspricht der des in Figur 1 gezeigten Gasaufbereiters 1. Im Unterschied zu dem in Figur 1 gezeigten Gasaufbereiter 1 strömt das Gas oder die Umgebungsluft im Betrieb des Gasaufbereiters 1 durch die Kanäle 7 und eventuell vorhandene Porositäten des Keramikbauteils 6 anstatt an den Zeolithteilchen 3 vorbeizuströmen.

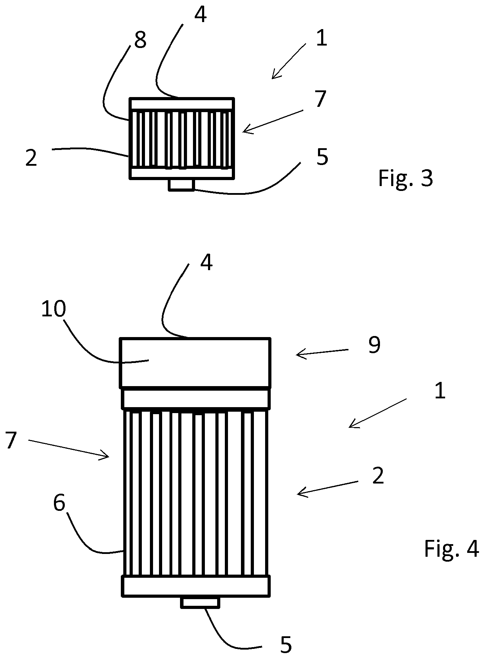

Figur 3 zeigt einen Gasaufbereiter 1 mit einem Wärmespeicher 2, der ein Metallbauteil 8 aufweist. Aufbau und Funktion entsprechen dem in Figur 2 gezeigten Wärmeaufbereiter 1. Wesentlicher Unterschied ist, dass der Wärmespeicher 2 durch die Verwendung des Metallbauteils induktiv aufgeladen werden kann.

Figur 4 zeigt einen Gasaufbereiter 1 mit einem Wärmespeicher 2 und einem Flüssigkeitsspeicher 9. Der Wärmespeicher 2 ist wie der in Figur 2 gezeigte Wärmespeicher 2 als Keramikbauteil 6 mit Kanälen 7 ausgebildet. Der Flüssigkeitsspeicher 9 ist stromaufwärts des Wärmespeichers 2 angeordnet und weist einen offenporigen Schwamm 10 auf.

Im Betrieb des Gasaufbereiters 1 strömt ein Gas durch die Gaszuführung 4 in den offenporigen Schwamm 10 und wird an der Oberfläche des Schwamms 10 mit Feuchtigkeit angereichert. Danach strömt das Gas durch die Kanäle 7 des Keramikbauteils 6 und wird an der Oberfläche der Kanäle 7 erwärmt. Das befeuchtete und erwärmte Gas strömt anschließend durch den Inhaliervorrichtungsanschluss 5 aus dem Gasaufbereiter heraus.

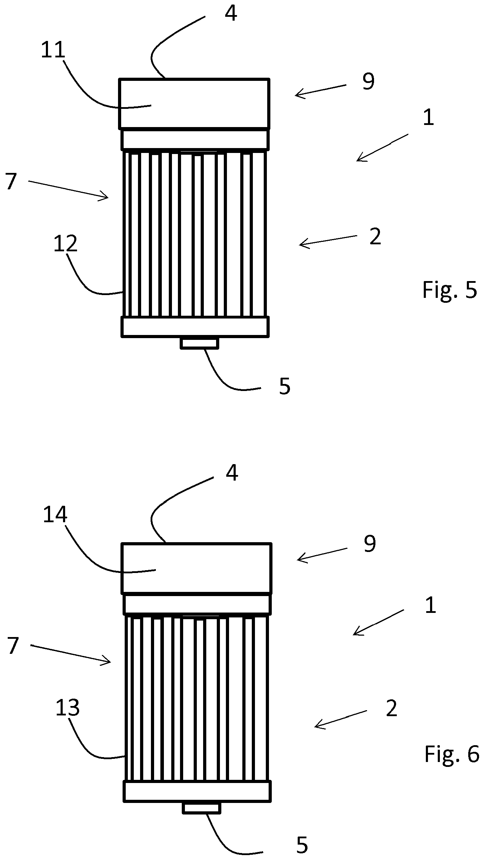

Figur 5 zeigt einen Gasaufbereiter 1 mit einem Wärmespeicher 2, der eine Flüssigkeit aufweist, und einem Flüssigkeitsspeicher 9. Aufbau und Funktion des Gasaufbereiters 1 ähneln dem in Figur 4 gezeigten Gasaufbereiter 1. Ein Unterschied ist, dass der Flüssigkeitsspeicher 9 statt des Schwamms 10 ein Tongranulat 11 aufweist. Das Tongranulat 11 wird im Betrieb des Gasaufbereiters 1 von Gas durchströmt und gibt an den Oberflächen der Tongranulatteilchen Feuchtigkeit an das Gas ab. Ein weiterer Unterschied ist, dass der Wärmespeicher 2 eine Flüssigkeit 12 aufweist, um Wärme zu speichern. Kanäle 7 sind durch die Flüssigkeit 12 geführt und ermöglichen ein Erwärmen des Gases durch die mit Wärme aufgeladene Flüssigkeit 12.

Figur 6 zeigt einen Gasaufbereiter 1 mit einem Wärmespeicher 2, der ein Metallbauteil 13 aufweist, und einem Flüssigkeitsspeicher 9. Aufbau und Funktion des Gasaufbereiters 1 ähneln dem in Figur 5 gezeigten Gasaufbereiter 1. Statt des Tongranulats 11 weist der Flüssigkeitsspeicher 9 Zellulose 14 auf. Die Zellulose 14 ist wie das Tongranulat 11 durchströmbar. Statt der Flüssigkeit 12 weist der Wärmespeicher 2 ein Metallbauteil 13 auf, das von Kanälen 7 durchzogen ist. Das Metallbauteil 13 kann induktiv mit Wärme aufgeladen werden.

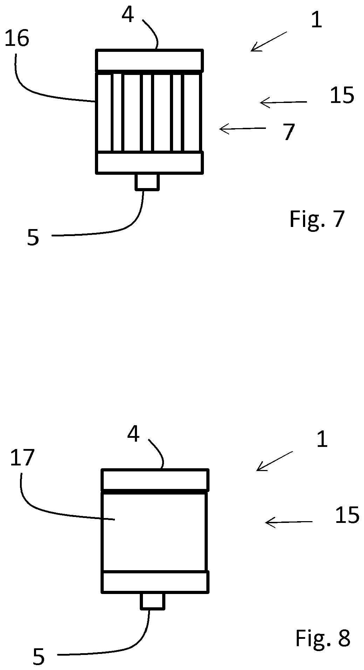

Figur 7 zeigt einen Gasaufbereiter 1 mit einem Kombinationsspeicher 15 für Wärme und Flüssigkeit, der ein Keramikbauteil 16 aufweist, das mit Kanälen 7 versehen ist. Das Keramikbauteil 16 ist dazu ausgelegt, sowohl Wärme als auch Flüssigkeit aufnehmen und abgeben zu können. Im Betrieb des Gasaufbereiters 1 strömt Umgebungsluft durch die Gaszuführung 4 in den Gasaufbereiter 1 hinein, nimmt an der Oberfläche des Keramikbauteils 16 sowohl Wärme als auch Feuchtigkeit auf, strömt durch die Kanäle 7 hindurch und tritt erwärmt und befeuchtet aus dem Inhaliervorrichtungsanschluss 5 heraus.

Figur 8 zeigt einen Gasaufbereiter 1 mit einem Kombinationsspeicher 15 für Wärme und Flüssigkeit, der Kieselgelgranulat 17 aufweist. Aufbau und Funktion ähneln dem in Figur 7 gezeigten Gasaufbereiter 1. Der Unterschied besteht darin, dass hier anstatt des Keramikbauteils 16 Kieselgelgranulat 17 als Wärme- und Flüssigkeitsspeicher eingesetzt ist. Das Kieselgelgranulat kann im Betrieb des Gasaufbereiters 1 von Gas durchströmt werden und Wärme und Feuchtigkeit an das Gas abgeben.

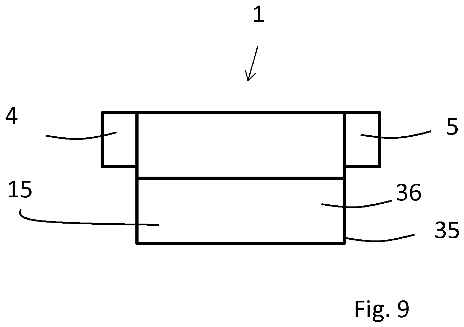

Figur 9 zeigt einen Gasaufbereiter 1 mit einem Kombinationsspeicher 15 für Wärme und Flüssigkeit, der Wasser 36 aufweist. Der Kombinationsspeicher 15 ist mit einem Wasserbehälter 35 ausgestattet, der dazu eingerichtet ist, Wasser 36 aufzunehmen.

Bei der Verwendung des Gasaufbereiters 1 wird warmes Wasser 36 in den Wasserbehälter 35 eingefüllt. Im Betrieb strömt Umgebungsluft durch die Gaszuführung 4 in den Gasaufbereiter 1 hinein, nimmt an der Oberfläche des Wassers 36 sowohl Wärme als auch Feuchtigkeit auf und tritt erwärmt und befeuchtet aus dem Inhaliervorrichtungsanschluss 5 heraus.

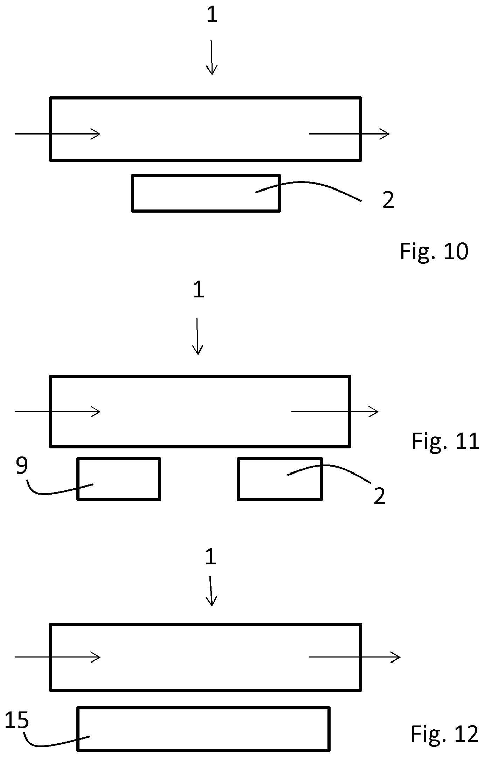

Figur 10 zeigt das Schema eines Gasaufbereiters 1 mit einem Wärmespeicher 2. Der Wärmespeicher 2 ist in Kontakt mit einem Gasstrom oder Einatemstrom. Zur Verwendung wird der Wärmespeicher 2 durch eine externe Quelle aufgeladen. Um die Wärme an den Einatemstrom zu übertragen, wird der Wärmespeicher 2 nach dem Aufladen von dem Einatemstrom umströmt, durchströmt oder sowohl umströmt als auch durchströmt. Um eine gute thermische Übertragung erreichen zu können, weist der Wärmespeicher 2 eine große Oberfläche auf, die es dem Einatemstrom ermöglicht, ausreichend Wärme pro Zeiteinheit aufzunehmen. Außerdem kann ein poröses Medium ausgewählt werden und derart ausgestaltet sein, dass es eine große Tortuosität aufweist, so dass die Verweilzeit eines Gases in dem porösen Medium verlängert oder optimiert wird. Der Einatemwiderstand wird dadurch leicht erhöht und der Wärmeübergang optimiert.

Die resultierende Temperatur der Einatemluft wird maßgeblich durch die Bauform, die Wärmekapazität und die Temperatur des Wärmespeichers bestimmt. Wenn der Gasaufbereiter 1 mit einem Düsenvernebler verwendet wird, wird das Gas, das den Gasaufbereiter 1 passiert hat, in dem Düsenvernebler mit einem Kompressorluftstrom gemischt, so dass die resultierende Aerosoltemperatur von den Temperaturen und dem Mischungsverhältnis dieser Gasströme abhängig ist. Temperaturverluste können durch Verdunstungskälte entstehen, die beim Verdunsten von Aerosoltröpfchen entsteht. Hierdurch kann die Aerosoltemperatur sinken und die Luftfeuchtigkeit auf 100 % gesteigert werden.

Figur 11 zeigt das Schema eines Gasaufbereiters 1 mit einem Wärmespeicher 2 und einem Flüssigkeitsspeicher 9. Das in Figur 10 gezeigte System kann mit einem Feuchtespeicher oder Flüssigkeitsspeicher 9 erweitert werden, um die angesaugte Atemluft gleichzeitig zu erwärmen und zu befeuchten. Die Atemluft kann auf 100 % Luftfeuchtigkeit gesättigt werden. So können Temperaturverluste durch Verdunstungskälte in einer angeschlossenen Inhaliervorrichtung minimiert und die Aerosoltemperatur maximiert werden.

Figur 12 zeigt das Schema eines Gasaufbereiters 1 mit einem kombinierten Wärme- und Flüssigkeitsspeicher 15. Hierdurch ergeben sich Vorteile in der Bauteiloptimierung und Handhabung. Hier ist ein poröses Medium mit verschiedenen Porösitäten und Permeabilitäten auf zwei Skalen gewählt. Auf der einen Skala ergibt sich eine Rückhaltesituation für Flüssigkeiten. Auf der anderen Skala stehen für die Gasphase perkolierende Pfade zur Verfügung.

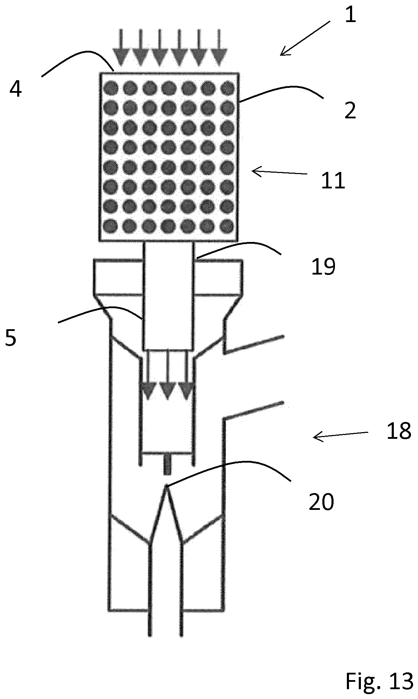

Figur 13 zeigt einen Düsenvernebler 18 mit einem Gasaufbereiter 1, der einen Wärmespeicher 2 aufweist. Der Wärmespeicher 2 weist Tongranulat 11 auf. Die Oberflächen der Tongranulatteilchen 11 sind als Wärmeübertragungsbereiche ausgebildet. Der Gasaufbereiter 1 weist eine Gaszuführung 4 aus, die als Vlies ausgebildet ist. Die Inhaliervorrichtungsanschluss 5 des Gasaufbereiters 1 ist mit einem Lufteinlass 19 des Düsenverneblers 18 verbunden.

Im Betrieb des Düsenverneblers 18 wird durch einen Einatemstrom Umgebungsluft durch die Gaszuführung 4 in das Innere des aufgeladenen Gasaufbereiters 1 gesaugt. Die Umgebungsluft wird über die Oberflächen der Tongranulatteilchen 11 erwärmt, strömt durch den Inhaliervorrichtungsanschluss 5 aus dem Gasaufbereiter 1 heraus und durch den Lufteinlass 19 des Düsenverneblers 18 in das Innere des Düsenverneblers 18 hinein. Die angesaugte Umgebungsluft strömt zusammen mit einem Kompressorluftstrom aus einer Patientenschnittstelle des Düsenverneblers 18 heraus und in nicht gezeigte Atemwege eines Anwenders hinein. Die Patientenschnittstelle ist in Figur 13 nicht gezeigt. Sie kann beispielsweise als Mundstück oder Nasenansatzstück ausgebildet sein.

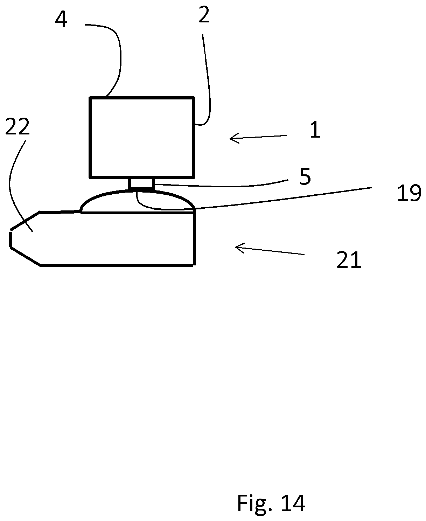

Figur 14 zeigt einen Meshvernebler 21 mit einem Gasaufbereiter 1, der einen Wärmespeicher 2 aufweist. Der Wärmespeicher 2 weist eine Gaszuführung 4 auf, die als Sieb ausgebildet ist. Im Betrieb des Meshverneblers 21 wird Umgebungsluft durch die Gaszuführung 4 hindurch in das Innere des Wärmespeichers 2 gesaugt, wo die Umgebungsluft angewärmt wird. Dies wird durch Wärmeübertragung mit einem der Wärmespeicher 2, wie sie beispielsweise in den Figuren 1 bis 3 beschrieben sind, erreicht. Durch den Inhaliervorrichtungsanschluss 5 wird die angewärmte Umgebungsluft aus dem Gasaufbereiter 1 heraus und in den Lufteinlass 19 des Meshverneblers 21 hinein gesaugt. Im Inneren des Meshverneblers 21 vermischt sich die aufbereitete Umgebungsluft mit von dem Meshvernebler 21 erzeugtem Aerosol und wird von einem Anwender durch das Mundstück 22 abgeatmet.

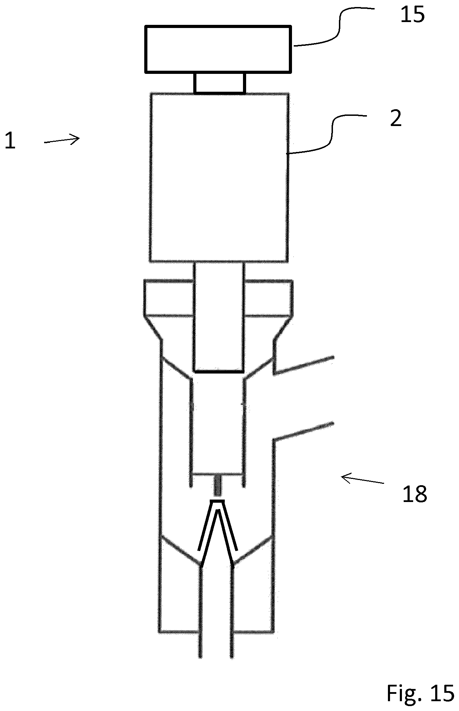

Figur 15 zeigt einen Düsenvernebler 18 mit einem Gasaufbereiter 1, der einen Wärmespeicher 2 und einen Flüssigkeitsspeicher 15 aufweist. Der Aufbau des Gasaufbereiters 1 entspricht beispielsweise einem der in den Figuren 4 bis 6 gezeigten Gasaufbereiter 1. Im Betrieb des Düsenverneblers 18 wird Umgebungsluft entsprechend dem in Figur 13 gezeigten Ablauf von dem Gasaufbereiter 1 aufbereitet, strömt durch den Düsenvernebler 18 und wird dem Anwender anschließend bereitgestellt. Im Unterschied zu dem in Figur 13 gezeigten Ablauf wird die Umgebungsluft bei der in Figur 15 gezeigten Vorrichtung auch angefeuchtet.

Durch die Befeuchtung weist die Einatemluft eine höhere Wärmekapazität auf. Eine Abkühlung durch Aerosolabtrocknung kann minimiert werden, da zum einen in dem feuchteren Gas eine geringere Aerosolabtrocknung stattfindet und zum anderen die Abkühlung durch die höhere Wärmekapazität geringer ist.

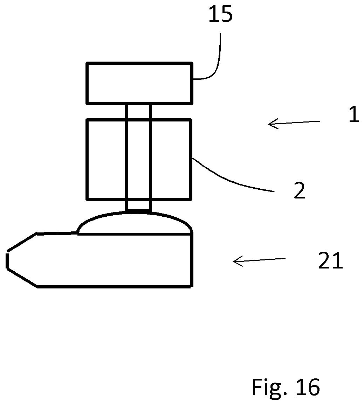

Figur 16 zeigt einen Meshvernebler 21 mit einem Gasaufbereiter 1, der einen Wärmespeicher 2 und einen Flüssigkeitsspeicher 15 aufweist. Der Aufbau des Gasaufbereiters 1 entspricht beispielsweise einem der in den Figuren 4 bis 6 gezeigten Gasaufbereitern 1. Im Betrieb des Meshverneblers 21 wird Umgebungsluft entsprechend dem in Figur 14 gezeigten Ablauf von dem Gasaufbereiter 1 aufbereitet, strömt durch den Meshvernebler 21 und wird dem Anwender anschließend bereitgestellt. Im Unterschied zu dem in Figur 14 gezeigten Ablauf wird die Umgebungsluft bei der in Figur 16 gezeigten Vorrichtung auch angefeuchtet.

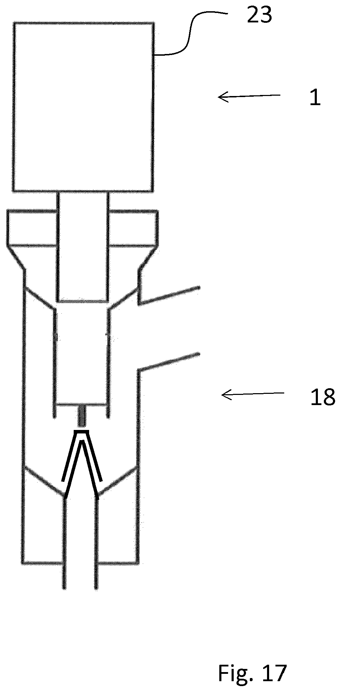

Figur 17 zeigt einen Düsenvernebler 18 mit einem Gasaufbereiter 1, der einen Kombinationsspeicher 23 für Wärme und Flüssigkeit aufweist. Der Aufbau des Gasaufbereiters 1 entspricht beispielsweise einem der in den Figuren 7 oder 8 gezeigten Gasaufbereitern 1. Die Funktion entspricht der in Figur 15 beschriebenen Funktion mit dem Unterschied, dass die Umgebungsluft nicht in zwei verschiedenen Bauteilen nacheinander angefeuchtet und erwärmt wird, sondern, dass dies gleichzeitig in dem Kombinationsspeicher 23 erfolgt.

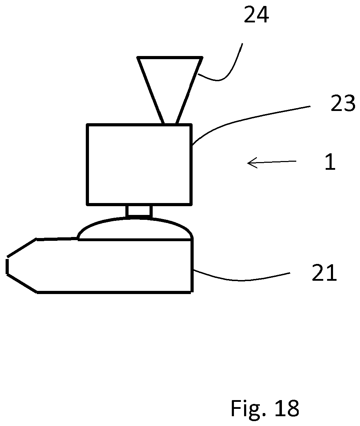

Figur 18 zeigt einen Meshvernebler 21 mit einem Gasaufbereiter 1, der einen Kombinationsspeicher 23 für Wärme und Flüssigkeit aufweist. Der Aufbau des Gasaufbereiters 1 entspricht beispielsweise einem der in den Figuren 7 oder 8 gezeigten Gasaufbereitern 1. Die Funktion entspricht der in Figur 16 beschriebenen Funktion mit dem Unterschied, dass die Umgebungsluft nicht in zwei verschiedenen Bauteilen nacheinander angefeuchtet und erwärmt wird, sondern, dass dies gleichzeitig in dem Kombinationsspeicher 23 erfolgt.

Zum Aufladen des Kombinationsspeichers 23 mit Flüssigkeit ist ein Trichter 24 vorgesehen, durch den eine Flüssigkeit in den Kombinationsspeicher 23 eingefüllt werden kann.

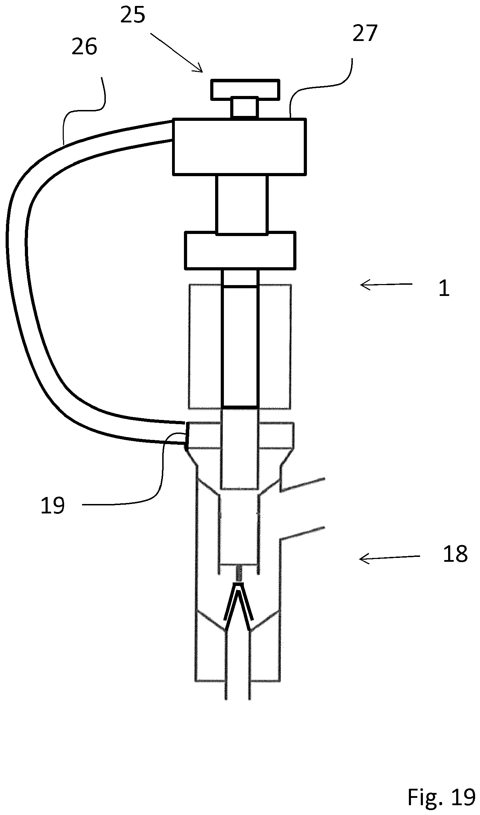

Figur 19 zeigt einen Düsenvernebler 18 mit einem Gasaufbereiter 1 und einer Temperatursteuerung 25. Düsenvernebler 18 und Gasaufbereiter 1 können beispielsweise aufgebaut sein, wie in Zusammenhang mit Figur 15 beschrieben. Die Temperatursteuerung 25 weist einen Bypass 26 und ein Ventil 27 auf. Der Bypass 26 ist mit dem Ventil 27 und einem Lufteinlass 19 durchströmbar verbunden. Der Gasaufbereiter 1 ist ebenfalls mit dem Ventil 27 und einem Lufteinlass 19 durchströmbar verbunden. Umgebungsluft, die den aufgeladenen Gasaufbereiter 1 passiert, wird befeuchtet und erwärmt. Umgebungsluft, die den Bypass 26 passiert, wird nicht aufbereitet. Mit dem Ventil 27 kann das Mischungsverhältnis dieser beiden Luftströme eingestellt werden. Durch Einstellung des Mischungsverhältnisses kann auch eine Temperatur des Aerosols eingestellt werden.

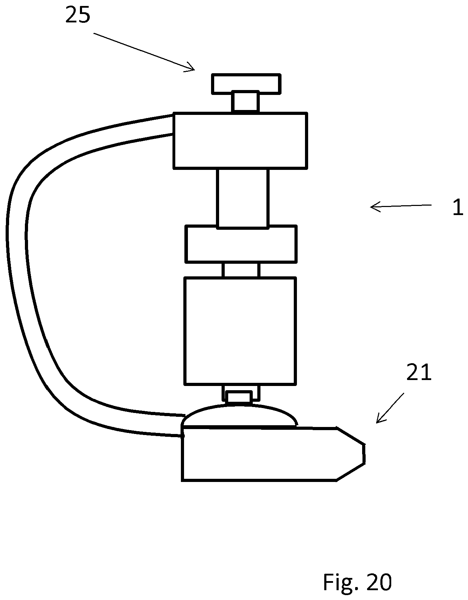

Figur 20 zeigt einen Meshvernebler 21 mit einem Gasaufbereiter 1 und einer Temperatursteuerung 25. Funktion und Aufbau entsprechen in Zusammenhang mit Figur 19 beschriebener Funktion und dem Aufbau. Der Unterschied des in Figur 20 gezeigten Systems zu dem in Figur 19 gezeigten System besteht darin, dass statt eines Düsenverneblers 18 ein Meshvernebler 21 eingesetzt ist.

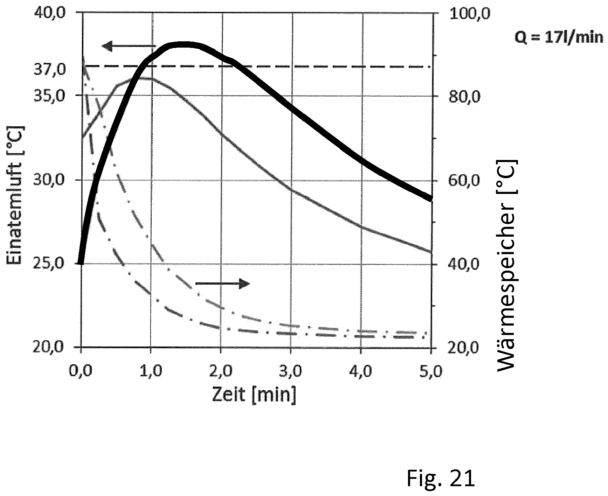

Figur 21 zeigt ein Diagramm mit Temperaturverläufen. Die beiden durchgezogenen Linien zeigen beispielhafte Verläufe der Aerosoltemperatur über der Zeit für eine anfängliche Wärmespeichertemperatur von ca. 90°C und einem Fluss von 17 l/min. Die dickere der beiden Linien zeigt den Verlauf für einen Wärmespeicher mit einer thermischen Masse von 40 g. Die dünnere der beiden Linien zeigt den Verlauf für einen Wärmespeicher mit einer thermischen Masse von 25 g. Die strichpunktierten Linien zeigen beispielhafte Temperaturverläufe von Wärmespeichern. Die obere der beiden strichpunktierten Linien zeigt einen Temperaturverlauf eines Wärmespeichers mit einer Masse von 40 g. Die untere der beiden strichpunktierten Linien zeigt einen Temperaturverlauf eines Wärmespeichers mit einer Masse von 25 g.

## Patentansprüche

1. Inhaliersystem mit einer Inhaliervorrichtung (18, 21) und einem Gasaufbereiter (1) für die Inhaliervorrichtung (18, 21),
wobei der Gasaufbereiter einen Gasaufbereitungsbereich, einen Wärmespeicher (2), eine Gaszuführung (4) und einen Inhaliervorrichtungsanschluss (5) aufweist, der Gasaufbereitungsbereich einen Wärmeübertragungsbereich aufweist,
der Wärmeübertragungsbereich mit dem Wärmespeicher (2) thermisch verbunden ist,
die Gaszuführung (4) dazu eingerichtet ist, ein Gas in den Gasaufbereitungsbereich zu führen, und
der Inhaliervorrichtungsanschluss (5) eine durchströmbare Verbindung zu dem Gasaufbereitungsbereich aufweist und durchströmbar mit einer Inhaliervorrichtung (18, 21) verbindbar oder verbunden ist,
wobei der Wärmespeicher dazu eingerichtet ist, in einer Aufladephase Energie von einer Ladestation aufzunehmen und in einer Inhalationsphase die in der Aufladephase aufgenommene Energie oder einen Teil davon in Form von Wärme abzugeben, und
wobei die Inhaliervorrichtung (18, 21) einen Gasaufbereiteranschluss (19) aufweist, der mit dem Inhaliervorrichtungsanschluss (5) durchströmbar verbindbar oder verbunden ist,
**dadurch gekennzeichnet, dass**
das Inhaliersystem einen Bypass (26) und eine Steuereinrichtung (25) aufweist, wobei der Bypass (26) dazu eingerichtet ist, eine Verbindung zwischen der Inhaliervorrichtung (18, 21) und einem Gasvolumen bereitzustellen und die Steuereinrichtung (25) dazu eingerichtet ist, ein Verhältnis zwischen einem Bypassströmungsquerschnitt und einem Gasaufbereiterströmungsquerschnitt einzustellen.

2. Inhaliersystem nach Anspruch 1, **dadurch gekennzeichnet, dass** der Gasaufbereiter (1) einen Flüssigkeitsspeicher (9) und der Gasaufbereitungsbereich einen Befeuchtungsbereich aufweist,
und der Flüssigkeitsspeicher (9) mit dem Befeuchtungsbereich verbunden ist.

3. Inhaliersystem nach Anspruch 2, **dadurch gekennzeichnet, dass** der Befeuchtungsbereich stromaufwärts des Wärmeübertragungsbereichs angeordnet ist.

4. Inhaliersystem nach Anspruch 2 oder 3, **dadurch gekennzeichnet, dass** der Flüssigkeitsspeicher (9) in den Wärmespeicher (2) integriert ist.

5. Inhaliersystem nach einem der vorhergehenden Ansprüche, **dadurch gekennzeichnet, dass** der Wärmespeicher (2) ein magnetisches Material aufweist.

6. Inhaliersystem nach einem der Ansprüche 2 bis 4, **dadurch gekennzeichnet, dass** der Wärmespeicher (2) ein poröses Material aufweist, der Flüssigkeitsspeicher (9) ein poröses Material aufweist oder sowohl der Wärmespeicher (2) als auch der Flüssigkeitsspeicher (9) ein poröses Material aufweisen.

7. Inhaliersystem nach einem der vorhergehenden Ansprüche, **dadurch gekennzeichnet, dass** das Inhaliersystem zum Erzeugen eines Aerosols eingerichtet ist und einen Temperatursensor umfasst, der dazu eingerichtet ist, eine Temperatur des Aerosols zu erfassen und ein Temperatursignal an die Steuereinrichtung (25) zu leiten, und die Steuereinrichtung (25) dazu eingerichtet ist, das Temperatursignal zu empfangen und basierend auf einer Solltemperatur und dem Temperatursignal ein Verhältnis zwischen Bypassströmungsquerschnitt und Gasaufbereiterströmungsquerschnitt einzustellen.

8. Verwendung eines Inhaliersystems mit einer Inhaliervorrichtung (18, 21) und einem Gasaufbereiter (1) nach einem der vorhergehenden Ansprüche zur Bereitstellung einer Vorrichtung für eine Warminhalation, wobei der Wärmespeicher (2) aufgeladen wird, und der Inhaliervorrichtungsanschluss (5) mit einem Gasaufbereiteranschluss (19) einer Inhaliervorrichtung (18, 21) verbunden wird.

## Claims

1. Inhalation system with an inhalation device (18, 21) and a gas preparation device (1) for the inhalation device (18, 21),
wherein the gas preparation device has a gas preparation region, a heat accumulator (2), a gas supply (4) and an inhalation device connection (5), the gas preparation region has a heat transmission region, the heat transmission region is thermally connected to the heat accumulator (2),
the gas supply (4) is configured to conduct a gas into the gas preparation region, and
the inhalation device connection (5) has a flowable connection to the gas preparation region and is connectable or connected to an inhalation device (18, 21), wherein the heat accumulator is configured to absorb energy from a charging station in a charging phase and to release the energy absorbed in the charging phase, or a part thereof, in the form of heat in an inhalation phase, and
wherein the inhalation device (18, 21) has a gas preparation device connection (19) which is connectable or connected to the inhalation device connection (5) in a flowable manner,
**characterised in that**
the inhalation system has a bypass (26) and a control device (25), wherein the bypass (26) is configured to provide a connection between the inhalation device (18, 21) and a gas volume and the control device (25) is configured to set a ratio between a bypass flow cross-section and a gas preparation device flow cross-section.

2. Inhalation system according to claim 1, **characterised in that** the gas preparation device (1) has a liquid reservoir (9) and the gas preparation region has a humidification region,
and the liquid reservoir (9) is connected to the humidification region.

3. Inhalation system according to claim 2, **characterised in that** the humidification region is arranged upstream of the heat transmission region.

4. Inhalation system according to claim 2 or 3, **characterised in that** the liquid reservoir (9) is integrated into the heat accumulator (2).

5. Inhalation system according to one of the preceding claims, **characterised in that** the heat accumulator (2) comprises a magnetic material.

6. Inhalation system according to one of the claims 2 to 4, **characterised in that** the heat accumulator (2) comprises a porous material, the liquid reservoir (9) comprises a porous material or both the heat accumulator (2) and the liquid reservoir (9) comprise a porous material.

7. Inhalation system according to one of the preceding claims, **characterised in that** the inhalation system is configured to generate an aerosol and includes a temperature sensor which is configured to sense a temperature of the aerosol and transmit a temperature signal to the control device (25), and the control device (25) is configured to receive the temperature signal and set a ratio between bypass flow cross-section and gas preparation device flow cross-section based on a setpoint temperature and the temperature signal.

8. Use of an inhalation system with an inhalation device (18, 21) and a gas preparation device (1) according to one of the preceding claims to provide a device for warm inhalation, wherein the heat accumulator (2) is charged, and the inhalation device connection (5) is connected to a gas preparation device connection (19) of an inhalation device (18, 21).

## Revendications

1. Système d'inhalation comprenant un dispositif d'inhalation (18, 21) et un conditionneur de gaz (1) destiné au dispositif d'inhalation (18, 21),
dans lequel le conditionneur de gaz présente une zone de conditionnement du gaz, un réservoir de chaleur (2), un système d'alimentation en gaz (4) et un raccordement de dispositif d'inhalation (5),
la zone de conditionnement du gaz présente une zone de transfert de chaleur, la zone de transfert de chaleur est reliée thermiquement au réservoir de chaleur (2),
le système d'alimentation en gaz (4) est conçu pour introduire un gaz dans la zone de conditionnement du gaz, et
le raccordement de dispositif d'inhalation (5) présente une liaison pouvant être traversée avec la zone de conditionnement du gaz et pouvant être reliée ou est reliée de manière à pouvoir être traversée avec un dispositif d'inhalation (18, 21),
dans lequel le réservoir de chaleur est conçu pour recevoir de l'énergie lors d'une phase de chargement d'une station de chargement et de délivrer lors d'une phase d'inhalation l'énergie reçue lors de la phase de chargement ou de délivrer une partie de celle-ci sous forme de chaleur, et
dans lequel le dispositif d'inhalation (18, 21) présente un raccordement de conditionneur de gaz (19), qui peut être relié ou est relié de manière à pouvoir être traversé au raccordement de dispositif d'inhalation (5),
**caractérisé en ce que**,
le système d'inhalation présente une dérivation (26) et un dispositif de commande (25), dans lequel la dérivation (26) est conçue pour fournir une liaison entre le dispositif d'inhalation (18, 21) et un volume de gaz et le dispositif de commande (25) est conçu pour établir un rapport entre la section transversale d'écoulement de la dérivation et une section transversale d'écoulement du conditionneur de gaz.

2. Système d'inhalation selon la revendication 1, **caractérisé en ce que** le conditionneur de gaz (1) présente un réservoir de liquide (9) et la zone de conditionnement du gaz une zone d'humidification,
et le réservoir de liquide (9) est relié à la zone d'humidification.

3. Système d'inhalation selon la revendication 2, **caractérisé en ce que** la zone d'humidification est disposée en amont de la zone de transfert de chaleur.

4. Système d'inhalation selon la revendication 2 ou 3, **caractérisé en ce que** le réservoir de liquide (9) est intégré au réservoir de chaleur (2).

5. Système d'inhalation selon l'une quelconque des revendications précédentes, **caractérisé en ce que** le réservoir de chaleur (2) présente un matériau magnétique.

6. Système d'inhalation selon l'une quelconque des revendications 2 à 4, **caractérisé en ce que** le réservoir de chaleur (2) présente un matériau poreux, le réservoir de liquide (9) présente un matériau poreux ou aussi bien le réservoir de chaleur (2) que le réservoir de liquide (9) présentent un matériau poreux.

7. Système d'inhalation selon l'une quelconque des revendications précédentes, **caractérisé en ce que** le système d'inhalation est conçu pour produire un aérosol et comprend un capteur de température, qui est conçu pour détecter une température de l'aérosol et transmettre un signal de température au dispositif de commande (25), et le dispositif de commande (25) est conçu pour recevoir le signal de température et pour établir un rapport entre la section transversale d'écoulement de la dérivation et la section transversale d'écoulement du conditionneur de gaz en s'appuyant sur une température de consigne.

8. Utilisation d'un système d'inhalation comprenant un dispositif d'inhalation (18, 21) et un conditionneur de gaz (1) selon l'une quelconque des revendications précédentes pour fournir un dispositif pour une inhalation chaude, dans laquelle le réservoir de chaleur (2) est chargé, et le raccordement de dispositif d'inhalation (5) est relié à un raccordement de conditionneur de gaz (19) d'un dispositif d'inhalation (18, 21).
